# EUROPEAN PATENT SPECIFICATION

(11) **EP 4 529 906 B1**
(45) Date of publication and mention of the grant of the patent: **14.10.2026**
(21) Application number: 24182955.5
(22) Date of filing: 18.06.2024
(51) Int. Cl.: A61F 13/00, A61F 13/01, A61L 15/32, A61L 15/64

(54) **SET FOR PRODUCING AN AUTOLOGOUS DRESSING FROM BLOOD, A DRESSING, METHOD OF PRODUCTION THEREOF**
SET ZUR HERSTELLUNG EINES AUTOLOGEN VERBANDS AUS BLUT, VERBAND, VERFAHREN ZU SEINER HERSTELLUNG
ENSEMBLE POUR PRODUIRE UN PANSEMENT AUTOLOGUE À PARTIR DE SANG, PANSEMENT, SON PROCÉDÉ DE PRODUCTION

(30) Priority: 30.09.2023 PL 44627923
(43) Date of publication of application: 02.04.2025
(73) Proprietor: Keymed Spólka z ograniczona odpowiedzialnoscia, 58-506 Jelenia Góra (PL)
(72) Inventor: Kasperek, Maksymilian, 58-506 Jelenia Góra (PL); Kasperek, Sylwia, 58-506 Jelenia Góra (PL)
(74) Representative: Grzelak, Anna

(56) References cited:
- US-A1- 2018 311 404

## Description

### TECHNICAL FIELD

An object of the invention is a set for producing an autologous dressing from whole blood of a subject, a method of production of an autologous wound dressing, an autologous dressing and the use thereof for the treatment of wounds and skin diseases. The set for producing the autologous dressing is used to prepare autologous tissue dressings, i.e. derived from the patient's own blood, accelerating the healing process of, i.a., burns, pressure sores, post-operative and ulcerative wounds. The invention is applicable in the field of medicine, health protection and veterinary medicine. The set for producing an autologous dressing can be used by health professionals for a wide range of elective or other procedures that may take place in health centers, treatment, nursing, medical or dental offices, which may take place in hospital facilities, but also first aid clinics, primary health care units, and even in remote places with limited infrastructure, such as war zones or rural areas.

### STATE OF ART

In recent years, there has been a significant development of biological products containing platelets used to treat difficult-to-heal wounds. The basic component of these products are platelets, which play an important role in the regulation of homeostasis and the formation of a fibrin clot. Platelets are responsible for the activation and release of important molecules, including platelet-specific proteins, growth factors, coagulation factors, adhesion molecules, cytokines, chemokines and angiogenic factors that participate in the stimulation of proliferation and activation of cells involved in wound healing such as fibroblasts, neutrophils, macrophages and mesenchymal stem cells (Eisinger et al., 2018).

There are also interactive dressings available on the market that perform not only a protective and securing function, but also support and stimulate the natural processes occurring in the healed wound, e.g. hydrogel, foam, fibrin, collagen or alginate dressings, as well as dressings that release substances with a specific therapeutic or antibacterial effect. There are many clinical studies that demonstrate the beneficial effects of growth factors on wound healing. In various clinical studies improvement in wound healing parameters after local application of factors such as: NGF, GM-CSF, b FGF and EGF was demonstrated. To date, however, the greatest hopes are associated with PDGF-BB, which has successfully passed all phases of clinical trials. Gels containing PDGF are already available, which, when applied to the wound, stimulate the healing of wounds caused by, e.g., diabetic neuropathy.

Attempts are also made to use platelet-rich preparations in the treatment of chronic wounds. Platelet-rich plasma is a concentrate of autologous growth factors, including: PDGF, IGF, VEGF, therefore it naturally stimulates both epidermal cells, as well as dermal fibroblasts and mesenchymal stem cells (MSCs), leading to the regeneration of the damaged tissue.

Hydrogel dressings are also known and have many advantages that traditional dressings do not have. Patches from a gel substance maintain the appropriate level of moisture, allow the skin to breathe, they are also transparent, which allows the observation of the wound healing process. Hydrogel has absorption properties, due to which it absorbs secretions with microorganisms oozing from the wound.

The document EP0998949A2 discloses an elastic wound dressing containing fibrin fibers, which is prepared from intensively dialyzed components or a completely free fibrinogen. The document also discloses a method of production of an elastic fibrin-based wound dressing, in which a fibrinogen solution is subjected to a single- or multi-step dialysis, and then, as a result of the action of the aqueous thrombin solution on the fibrinogen solution, an elastic fibrin fabric is formed, which is then subjected to freeze-drying.

The document WO2014209620A1 discloses a fibrin-coated wound care product having a flexible film layer, a pressure-sensitive adhesive layer placed on the flexible film layer, and a fibrin powder layer placed on the surface of the pressure-sensitive adhesive layer, placed opposite of the flexible film layer. The document also discloses a method of production of a wound care article, including the steps of: providing a flexible film layer, placing a pressure-sensitive adhesive layer on the flexible film layer, forming a layer of fibrin powder on the surface of the pressure-sensitive adhesive layer opposite the flexible film layer, in order to prepare the article for dressing wounds.

The publication US2018311404 discloses a device for preparing an autologous fibrin biological dressing, which consists of a device comprising a cylindrical container, which bottom has side inclinations α, leading to a central channel with a slope β that directs the liquid sealant created by forcing the fibrin coagulate through the sieve towards the outlet nozzle and then towards the multichannel collection system. According to the disclosure, the already centrifuged blood in the form of a clot is introduced into the device onto a sieve, and pressure is applied to the fibrin clot to shape the clot. The disadvantage is that mechanical pressure destroys the cells contained in the clot, affecting the biological properties and regenerative abilities of the blood dressing obtained by using the device. The document does not disclose a method of production of an autologous wound dressing.

A method of production of the autologous ActiGraft^{®} System dressing is described (https://www.hmpgloballearningnetwork.com/site/wounds/original-research/safety-and-efficacy-autologous-blood-clot-product-management-texas-1a), in which the patient's whole blood is collected using a citrate anticoagulant, which inhibits the blood clotting cascade. Subsequently, to form a clot, blood clotting is reactivated in a plastic disposable container by mixing it with a suspension of calcium gluconate and kaolin powder. Unfortunately, the clot produced in this way does not reproduce all the biological and regenerative properties, which are limited by adding an agent that inhibits clotting and then artificially activating clotting by adding agents that activate the process.

From the publication by Ghanaati et al., 2014, a method of production of an autologous dressing from whole blood is known. To obtain the cellular fibrin fraction (A-PRF), the blood sample is centrifuged at 1500 RPM for 14 minutes. After centrifugation, the clots are carefully extracted from the test tubes, the red blood cell (RBC) fraction is removed in such a way as not to damage the bottom of the fibrin-rich clot. Fibrin-based clots with a buffy coat and some part of the red blood cells are fixed with a 4% paraformaldehyde solution for 24 hours. Subsequently, they are placed along the longitudinal axis in the mounting cassettes. By pressing the clots into the embedding cassette, a dressing with dimensions of approx. 3.5 cm long x 1 cm wide x 0.1 cm thick is obtained from 10 ml of whole blood. The disadvantage of the described method is the low efficiency of the surface and volume of the dressing from 1 ml of whole blood, and the dressing is in an inconsistent form, moreover the mechanical pressure destroys the cells contained in the clot, affecting the biological properties and regenerative abilities of the autologous dressing from blood, obtained using the given method. In the dressing, the remains of paraformaldehyde used during the production remain.

It is estimated that approximately 1.5% of citizens of developed countries are affected by the problem of a chronic, difficult-to-heal wound during their lives. Suffering, pain, unpleasant odor, the risk of infection and high costs associated with the purchase of dressings, anti-bedsore mattresses and rehabilitation affect the quality of life of patients with chronic wounds and their caregivers and families. The sick persons, affected by the problem of chronic wounds require professional care in the field of wound care, including, i.a., frequent replacement of specialized dressings, which is why they need regularly visit primary care physicians, outpatient specialist offices and often require frequent hospitalization. This constitutes a serious social, medical and economic problem. The problem of treating difficult-to-heal and chronic wounds necessitates the search for new, more effective methods of therapy. Currently, mainly sterile or non-sterile dressings and fabric bandages, which do not accelerate the healing process, have a short shelf life and may cause allergic reactions due to the content of synthetic components, are used to treat and supply wounds. Traditional dressings cause pain during hygiene activities, especially when they need to be changed frequently. The commonly used dressings are easily soiled by clothes, and their edges peel off spontaneously relatively easily. A cotton gauze dressing does not keep the wound warm, is not waterproof and does not allow for bathing or hydrotherapy. Wetting the dressing requires changing it each time, as it could aggravate the undesirable maceration of the skin around the wound. A traditional dressing also does not ensure joint mobility and causes tension of the skin surrounding the wound during movement, forcing the dressing to be applied to an already bent limb.

Burns are one of the most serious body injuries. In most cases, their nature is sudden, severely damaging tissues, and even in a small area, they lead to deep and extensive damage. Children and the elderly are particularly vulnerable to burns. Burns to children under seven years of age account for one quarter of all burns. Throughout the world, burns are a very serious public health problem, both in terms of physical, psychological and economic consequences. Each year, about 1% of the population suffers from various types of burns. The pain, disfigurement and body deformations associated with burns require long-term specialist treatment. Systemic disorders called burn disease directly threaten the lives of adults, as well as children. These accidents require the implementation of highly specialized, intensive general and local treatment. Treatment of burns mainly involves fighting the infection, replenishing blood and protein, and covering the burnt surfaces with appropriate skin grafts. Sterile dressings and frequent wound cleaning are used, but this is a long-term therapy. Another technical problem is providing appropriate dressings for people with pressure sores. The methods currently used are invasive, cause pain and suffering to patients, and lengthen the recovery period. An appropriate dressing for people with pressure sores should minimize pain, reduce the invasiveness of the treatment and shorten the recovery period. Pressure sores are a serious social problem because as societies age, they affect an increasingly wider group of people.

Therefore, in the state of the art, there is no solution dedicated to obtaining an autologous dressing tailored to the wound, in terms of providing a set for producing an autologous dressing, for easy production of an autologous dressing from material derived from the patient's blood, tailored to the size and shape of the wound, or an easy method of obtaining an autologous dressing, from a material derived from the blood of the patient, for whom the dressing is intended. To date, no complete product for easy obtaining of tailored autologous dressings, i.e. dressings obtained directly from the patient's blood, which would enable the selection of its size according to the size of the wound, has been disclosed. No method of production or sets dedicated to this method for obtaining autologous dressings was also developed, especially for obtaining a dressing from two fractions of the patient's blood, of a different size and shape, appropriately tailored to the size of the wound, which, if applied directly to the patient's wound, would accelerate the healing process not only due to the composition (constitution) of the dressing, but also due to its proper fit to the wound and gel consistency. Therefore, in the state of the art there are no sets for producing autologous dressings for the treatment of wounds of various sizes, tailored to the shape and extent of the wound, which will enable tailoring of the dressing to the wound, thus improving the treatment process, accelerating wound healing, reducing scarring, and minimizing pain and allergic reactions when changing dressings or using dressings containing substances that may cause allergic reactions.

### Essence of the invention

The aim of the invention is to provide a simple and easy-to-use set for producing an autologous dressing, enabling the production of an autologous wound dressing by a simple method in various medical care facilities, which can be produced in various shapes and sizes, and thus provide the ability to tailor the size of the dressing to a given wound, and the dressing produced by this method, which overcomes the disadvantages of the state of the art. The developed solution according to the invention is intended to provide a simple to handle and to use set for producing an autologous dressing, easy to use in all conditions, including repeated use, even in a primary medical care facility, allowing for the tailoring of the size and depth of the dressing to the wound surface, reducing the pain associated with changing of the dressing compared to the changing of the standard dressings, no need for frequent changing of dressings compared to the changing of standard dressings, natural absorption of the dressing, preventing bacterial infections of wounds, preventing unpleasant odor in the wound, accelerating wound healing. The aim of the invention is also to provide an easy method of production of an autologous dressing from the subject's own blood, the dressing produced by this method, and to provide a method of treating skin wounds using an autologous dressing from the subject's blood.

The invention relates to a set for producing an autologous dressing from blood, which comprises a shaping container and a cover of the shaping container with a shape and size resulting from the n- series of types; the shaping container has a substantially flat base and side walls of the shaping container of substantially equal height; the cover has a lid and side walls of the cover of substantially equal height; the cover is matched in shape and size to the shaping container so that when the cover is placed on the shaping container, the side walls of the cover substantially adhere to the inner side to the outer side of the side walls of the shaping container; the side walls of the shaping container are higher than the side walls of the cover, the shaping container is made of stainless steel and at least the bottom surface of the shaping container has a rough surface from the inside thereof, with a roughness degree in the range of 0.01-100 µm.

In the preferred set, both the shaping container and the cover of the shaping container are made of stainless steel, preferably of stainless steel approved for medical use.

In the preferred set, at least one hole and/or inlet for insertion is provided in the cover.

In the preferred set, the inlet for insertion is produced as a luer-lock or luer type.

In the preferred set, the shaping container and the cover of the shaping container in the n-series of types are selected from a shape with a cross-section with a shape similar to a square, circle, ellipse, rectangle, triangle, hexagon, heart shape, preferably a square with rounded vertices.

In a preferred set the roughness degree is in the range of 0.05-10 µm, more preferably in the range of 0.2-0.4 µm.

In a preferred set, the rough surface is a surface of the sandblasted type, diamond dust type, a surface with linear porosity, a surface with cross-porosity.

In a preferred set, the rough surface is produced by sandblasting, by producing micro-cuts and/or micro-dimples, preferably the micro-cuts and/or micro-dimples are produced by laser or mechanically.

The preferred set has a cross-sectional shape similar to a square with rounded vertices, an external size of 52x52±20% mm, a side wall (8) of the cover with a height of 10±20% mm; in the cover (2) there are four evenly spaced holes (4) and a central inlet for insertion (3) of the luer-lock type, wherein both the shaping container (1) and the cover (2) are made of stainless steel, wherein the inner surface of the bottom (9) of the shaping container (1) has a rough structure in the range from 0.2 to 0.4 micrometers, more preferably, the entire inner surface of the shaping container (1) has a rough structure with a roughness degree from 0.2 to 0.4 µm.

The invention also relates to a method of production of an autologous dressing from the blood of a subject, comprising the steps in which a) the blood collected from the subject is separated into two volumes, preferably two substantially equal volumes; from the first volume, the cell-rich fibrin fraction (I-PRF) is obtained by centrifugation from 50 RCF (xg) to 70 RCF (xg) for 3 to 5 minutes; from the second volume, the S-PRF structural fibrin fraction is obtained by centrifugation at 600 RCF (xg) to 800 RCF (xg) for 8 to 16 minutes; b) the I-PRF and S-PRF fractions obtained in a) are introduced into the shaping container (6) of the set for producing autologous tissue dressings from blood of the invention; wherein the I-PRF and S-PRF fractions are combined in a ratio of 1:5±20% of the I-PRF fraction to the S-PRF fraction; c) to produce an autologous dressing, the set for producing autologous tissue dressings with combined I-PRF and S-PRF fractions is set aside for the time needed to make the gel form, preferably for 5 to 240 minutes.

In the preferred method in step c), the set for producing autologous tissue dressings with combined I-PRF and S-PRF fractions is set aside for a time in the range of 10-40 min, more preferably in the range of 15-30 minutes.

In the preferred method in step c), before the set is set aside to produce an autologous tissue dressing, the combined I-PRF and S-PRF fractions are gently mixed together preferably by gentle rocking the shaping container (6) of the set.

In the preferred method in step b) the I-PRF and S-PRF fractions are combined in a ratio of 1.5:4±20% of the I-PRF fraction to the S-PRF fraction.

In the preferred method in step b) the I-PRF and S-PRF fractions are combined in a syringe, preferably in a syringe equipped with a luer-lock type tip.

In the preferred method, to produce 1 cm³ of an autologous dressing with a thickness of 1±10% mm, 2.1-2.3 ml, more preferably 2.15-2.25 ml of the mixture of the combined I-PRF and S-PRF fractions is introduced into the shaping container (6) of the set.

In the preferred method in step a), the separation to the cell-rich fibrin fraction (I-PRF) and the structural fibrin fraction (S-PRF) is carried out by parallel centrifugation in two centrifuges.

In the preferred method in step b) the combined I-PRF and S-PRF fractions are introduced into the shaping container (6) through the hole (4) in the cover (2) of the set for producing autologous tissue dressings and/or through the inlet for insertion (3) in the cover (2), preferably introduced through the inlet for insertion (3), preferably introduced through the luer, luer-lock type inlet.

In the preferred method in step a) the separation into the cell-rich fibrin fraction (I-PRF) and the structural fibrin fraction (S-PRF) is carried out by centrifugation of the blood in apyrogenic tubes.

In the preferred method the subject is a mammal, preferably a human.

The invention also relates to an autologous dressing from blood prepared by the method of the invention.

The autologous dressing from blood according to the invention and/or produced by a method according to the invention is for use in the treatment of a wound, a diabetic wound, a diabetic foot wound, a burn wound, a pressure sore wound, a frostbite wound, an ulcerative wound, a traumatic wound, a surgical wound, a chronic wound, a cut wound, a stab wound, a crush wound, a wound resulting from the action of radiant energy, a wound after radiological therapy, a wound after chemical trauma, a postoperative wound, a cut of the skin, an abrasion of the skin, a scratch of the skin, an inflammation of the skin, a rosacea.

The autologous dressing from blood is preferably used to treat a diabetic wound, diabetic foot wound, burn wound, pressure sore wound, frostbite wound, ulcerative wound, postoperative wound.

The autologous dressing from blood is changed preferably at least every seven days during the treatment.

The autologous dressing from blood is changed preferably every 3-4 days during the treatment, preferably in a subject when used to treat an ulcerative wound, a pressure sore wound.

The autologous dressing from blood is preferably used in a subject that is a mammal, preferably a human.

The invention therefore relates to a new and easy-to-manufacture method of production of an autologous dressing using a set for producing an autologous dressing from blood for producing autologous tissue dressings, i.e. from the patient's own blood, which accelerates the healing process of, i.a., burn wounds, pressure sores, post-operative and ulcerative wounds and skin diseases.

The method of production of the dressing according to the invention is based on obtaining two fractions of fibrin from the patient's own blood: the cell-rich fibrin fraction (I-PRF), containing a concentrated amount of platelets, leukocytes, cytokines, MSC and CD 34+ stem cells, and the structural fibrin fraction (S-PRF) with fibrin, then both fractions are combined to obtain an autologous tissue dressing, preferably using a set for producing autologous tissue dressings of the invention. The main aim achieved according to the disclosed invention is to develop an autologous dressing that will shorten the wound healing time by approximately 50% of the time currently needed to heal wounds, as well as to reduce treatment costs. Additional benefits achieved using an autologous dressing made from the patient's own blood obtained according to the invention include reducing scarring and minimizing pain and the possibility of allergic reactions compared to standard dressings.

The method of production of the dressing according to the invention, due to the separate centrifugation of the two fibrin fractions at low speed and the lack of mechanical pressure on the clot to form it, unlike the known methods, minimizes the process of destruction of cells and blood factors that, unchanged, may participate in the coagulation cascade and production of the autologous dressing.

Unlike the previously known methods, no additional substances or additives are used to produce an autologous dressing according to the invention, and the autologous dressing according to the invention is created as a result of reproducing the natural and physiological process of blood coagulation.

During many experiments performed with tests of various types of materials for the production of a shaping container (plastics, laboratory plates), even when producing a dressing from a cell-rich fibrin fraction (I-PRF) and a structural fibrin fraction (S-PRF) according to the disclosed method, results were not as good as when the shaping container was made of metal, preferably stainless steel, with at least a rough inner bottom surface of the shaping container. Otherwise, clotting was quite slow and the resulting clot did not have such a uniform gel consistency with a substantially uniform thickness over the surface of the entire dressing produced. Many experiments with tests were carried out, during which centrifugation parameters, clotting times of the autologous dressing, durability of the dressing and the ease of separating from the inner bottom surface of the shaping container were compared depending on the type of material from which the shaping container was made. In the case of tests with plastic containers and containers made of medical stainless steel with a completely smooth inner surface - the dressing remained semi-liquid, not gel-like. This is probably why, in previously known methods, coagulation activating substances are usually added, but in the case of the shaping container of the present disclosure there is no such need.

Without being bound by any theory, it is the roughness of the internal surface of the shaping container that activates the adhesion of platelet cells from the obtained cell-rich fibrin I-PRF and structural fibrin S-PRF to the surface of the shaping container, thanks to which the cellular material quickly polymerizes and creates an autologous dressing with a gel consistency. The activated dressing clotting process takes place much faster than on the other tested surfaces. Unexpectedly, it turned out that the porous surface inside the container accelerates and properly structures the production of the dressing, which leads to the production of a gel form of dressing. In addition, it ensures the appropriate structure of the produced dressing, which results in a dressing of substantially uniform thickness shaped according to the form of the shaping container used. The roughness at the bottom of the shaping container most likely allows for adhesion - the adhesion of platelets, which then activates and triggers the next steps of the coagulation cascade. Experiments have shown that the pH of steel has no effect on stopping the coagulation process and producing an autologous gel dressing. The form of autologous dressing obtained according to the invention enables easy application and adjustment of the dressing to the wound, and also influences its therapeutic properties.

The set for producing autologous tissue dressings is made of stainless steel intended for medical use. In the shaping container, at least the bottom surface of the shaping container has a rough surface on the inside, with a roughness degree in the range of 0.01-100 µm. The rough surface may be of the sandblasted type, diamond dust type, and may be in the form of linear or crossshaped porosity, for example produced as a result of cuts or recesses, e.g. cuts made by a laser, or any other mechanical method. Preferably, for example, the rough surface is obtained during the sandblasting process, thanks to which a rough surface in the range of 0.05-10 µm, more preferably in the range of 0.2-0.4 µm can be obtained.

In one embodiment, the cover of the set for producing autologous tissue dressings may not have any holes and the set can simply be sterilized for longer, at a higher temperature or as two separate parts, i.e. the shaping container and the top cover, separately. In such a case, the cellular material for the production of an autologous dressing is poured directly into the shaping container and then a cover adapted in shape and size is applied, trying to carry out the process in sterile conditions.

In one embodiment, the cover of the set for producing autologous tissue dressings has an inlet for insertion, preferably of the luer-lock or luer type, which serves to inject autologous material into the set and also facilitates sterilization of the set.

In the preferred embodiment of the set, the upper cover has a hole or holes useful for easier sterilization of the set (the holes ensure quick and even equalization of pressure and temperature inside the set during the sterilization process). In the case of a small size container, the inlet for insertion is also a hole, ensuring easy sterilization.

One of the advantages of the described solution is the proposed approach not only in the form of an optimized method of production of an autologous dressing, but also adapting the size and shape of the dressing to the wound, using a dedicated set for producing an autologous dressing, which ensures even and fairly tight coverage of the entire wound surface and accelerates its healing process.

The gel consistency of the obtained dressing allows it to adapt to the shape of the wound. Due to its consistency, the dressing completely fills the wound space and stops the access of bacteria, viruses and other microorganisms from the external environment. Cells present in the dressing: leukocytes, cytokines, platelets, growth factors, hematopoietic and mesenchymal stem cells, stimulate the accelerated process of wound healing and tissue regeneration. Autologous dressing works on a similar principle to tissue graft. When applied to the wound, it constitutes a matrix for the newly formed tissue, completely integrating into the wound space.

To date, no sets have been developed for the production of autologous dressings for obtaining autologous dressings from the patient's own blood of various sizes, appropriately tailored to the size of the wound, which, when applied directly to the patient's wound, would accelerate the healing process.

The set for producing an autologous dressing, which is the object of this application, overcomes the disadvantages of the state of the art by providing the possibility of producing an autologous dressing easily and tailored to the wound, which significantly simplifies and accelerates the treatment process, minimizing pain and disfigurement. Moreover, the present invention allows for the production of an autologous dressing that enables the treatment of difficult-to-heal chronic wounds, such as pressure sores, arterial and venous leg ulcers, and burn and post-operative wounds. The set for producing an autologous dressing from the patient's blood has the size and shape resulting from the n-series of types, which makes it possible to produce a dressing adapted to the size/extent of the wound, as well as the depth of the wound. The autologous dressing produced according to the invention accelerates wound healing and prevents wound complications by ensuring appropriate humidity, pH, gas exchange and thermoregulation, as well as the controlled oozing from the wound, without the need for frequent dressing replacement, as is the case with the known material or hydrogel dressings. The autologous dressing produced according to the invention eliminates the risk of bacterial infections and allergic reactions. Unlike the known dressings, it does not limit joint mobility and thus enables physical activity necessary for rehabilitation.

The set for producing an autologous dressing includes a shaping container and a cover matched both in shape and size. The shaping container has a substantially flat base and side walls of substantially equal height. The cover has a lid of the cover and side walls of the cover of substantially equal height. The cover is tailored to fit the shaping container so that when the cover is placed on the shaping container, the side walls of the cover adhere with their inner side to the outside of the side walls of the shaping container. The height of the side walls of the shaping container is higher than the height of the side walls of the cover, so that when the cover is placed on the shaping container, the side walls of the shaping container are not completely covered by the side walls of the cover. The cover has at least one hole and an inlet for insertion, enabling direct introduction of the patient's autologous material into the assembled set, e.g. using a syringe. Preferably, the inlet for insertion is of the luer-lock type, which enables easy introduction of the patient's autologous material from a syringe with a luer-lock tip screwed onto the luer-lock end. The shaping container and the cover, matched both in shape and size, may have a cross-sectional shape similar to a square, rectangle, ellipse, circle, heart, triangle, pentagon, hexagon or other polygon and made in the size resulting from the n-series of types.

Various sizes and shapes of the set allow the production of an autologous dressing with a shape and size similar to the wound to which the dressing is to be applied.

It is advantageous when the set includes a shaping container and a cover matched both in shape and size with a polygonal cross-section, e.g. essentially a square or rectangle, and the vertices are rounded, which prevents the dressing from being torn off or fragmented after its production while removing it from the shaping container.

For example, a set for producing an autologous dressing includes a shaping container and a cover, matched both in shape and size, with a substantially square cross-section with rounded vertices in the range of 1-25 cm, the length of each side wall of the shaping container and the height of the side wall of the shaping container in the range of 0.2 cm-10 cm, e.g. with a size of 5 cm x 5 cm x 2 cm, 7.5 cm x 7.5 cm x 3 cm, 10 cm x 10 cm x 4.

For example, a set for producing an autologous dressing includes a shaping container and a cover with a circular cross-section in the diameter range of 1-30 cm and the height of the side wall of the shaping container in the range of 0.2-10 cm. For example, with a diameter of 2, 3, 4, 5, 6, 7, 8, 9, 10, 11, 12, 13, 14, 15, 16, 17, 18, 19, 20, 21, 22, 23, 24, 25 cm.

A set for producing an autologous dressing of various shapes and sizes consistent with the n-series of types of the wound being dressed and the autologous dressing produced using it according to the described method is the answer to problems related to the healing of difficult-to-heal wounds, especially burns and pressure sores.

Various designs of the sets are possible, but at least the shaping container is made of metal, preferably stainless steel, preferably stainless steel approved for use in medicine and veterinary medicine. The cover is preferably made of the same type of material, but it is permissible to make it of plastic, preferably transparent.

The set is intended to be reusable and sterilizable.

Therefore, when a set for producing autologous dressings is intended for repeated use, most often the entire set is made of a metal material, most often stainless steel, and is sterilizable. The set can be easily washed, sterilized and reused. The ease and effectiveness of sterilization and venting when feeding the material to be produced into the assembled set is ensured by at least one hole in the cover.

Dressings produced from autologous platelet-rich fibrin according to the method of the invention will enable wound treatment using autologous platelets, growth factors, stem cells obtained from the patient's own blood. The process of producing a dressing involves obtaining two fractions of fibrin from the patient's own blood: the cell-rich fibrin fraction (I-PRF) and the structural fibrin fraction (S-PRF). Then both fractions are combined to obtain an autologous tissue dressing. Preferably, a set for producing an autologous dressing is used to produce the dressing, tailored to the wound both in shape and size consistent with the n-series of types of the wound being dressed. Autologous dressing provides, i.a.:
- acceleration of wound healing by approximately 50%;
- reduction in the frequency of dressing changes: once every 7 days (compared to regular dressings, which are initially changed every day, then approximately every 2-3 days, and wherein this activity does not cause pain, unlike changing a regular dressing);
- undergoes complete absorption;
- ensures safe use (due to the use of autologous material, the risk of an allergic reaction is eliminated).

It turned out that the autologous dressing according to the invention is incorporated into the wound tissue to which it is applied. Individual cellular elements of fibrin: leukocytes, cytokines, platelets, growth factors, hematopoietic and mesenchymal stem cells stimulate the accelerated process of wound healing and tissue regeneration. Platelets release growth factors. They undergo selfdegradation within 7 days through natural physiological processes. The growth factors present in the dressing stimulate repair processes, including: angiogenesis, fibroblast proliferation, epidermal cell proliferation, increase in collagen synthesis, increase in the synthesis of extracellular matrix proteins, and migration of mesenchymal stem cells. Leukocytes stimulate the migration of macrophages from connective tissue. Macrophages are responsible for cleansing the tissue of cellular debris, which enables the process of tissue regeneration and reconstruction to begin. Hematopoietic stem cells have the ability to divide indefinitely and differentiate into cells of other tissues. Gradually depolymerizing fibrin serves as a temporary matrix for the newly forming tissue. The dressing therefore acts as an autologous tissue graft.

The conducted clinical trials demonstrated high effectiveness of the autologous dressing produced according to the method of production of the dressing according to the invention, in the set for producing the dressing according to the invention, in various types of wounds in patients with various diseases, including: in groups of patients with ulcerative, pressure sore, burn and postoperative wounds and diabetic foot. In all cases examined, the use of the dressing according to the invention resulted in a significant reduction in the length, width and depth of the wounds. The effectiveness of the dressing was confirmed even in wounds that had been present in patients for a very long time, e.g., the healing process of a pressure sore wound that had not undergone any treatment for 10 years was achieved. The autologous dressing works not only on shallow wounds, but also on deep wounds, even up to 100 mm deep. In the conducted research, the effective operation of the dressing was demonstrated in a patient with a pressure sore wound on the buttock, which decreased from a depth of 70 mm to the depth of 50 mm (reduction of over 30%) just in two weeks after the start of therapy (results not shown).

When using the dressing according to the invention, the dressing was usually changed once every 7 days, which was completely sufficient and did not disturb the wound healing process. However, it was observed that in patients with large and extensive ulcerative and pressure sore wounds, which are characterized by high exudate, it is beneficial to change the dressing more often, e.g. every 3-4 days, which in turn additionally reduced the risk of wound oozing and tissue maceration and further accelerated the healing process.

Due to the above effects, the autologous dressing according to the invention is an excellent alternative to the currently used dressings and significantly simplifies and shortens the procedure of treating difficult-to-heal wounds, and thus increases the effectiveness of treatment and the comfort of patients' lives. At the same time, the use of an autologous dressing reduces the costs of wound treatment. For example, the current cost of treating a burn wound is estimated at 2 thousands up to 58 thousands PLN. The cost of a hospital stay for a patient with a pressure sore is estimated at approx. 120 PLN per day, and treatment may last up to several dozen months. Additionally, pressure sores usually destroy the expenditure incurred on rehabilitation, conservative and surgical treatment. The resulting autologous dressing according to the invention accelerates the healing process of wounds, including burns, pressure sores, ulcers, diabetic wounds and postoperative wounds, by 50%, and thus, in addition to increased effectiveness, reduces the costs of their treatment.

### BRIEF DESCRIPTION OF DRAWINGS

Examples of embodiments of the invention are presented in the figures of drawing, in which:

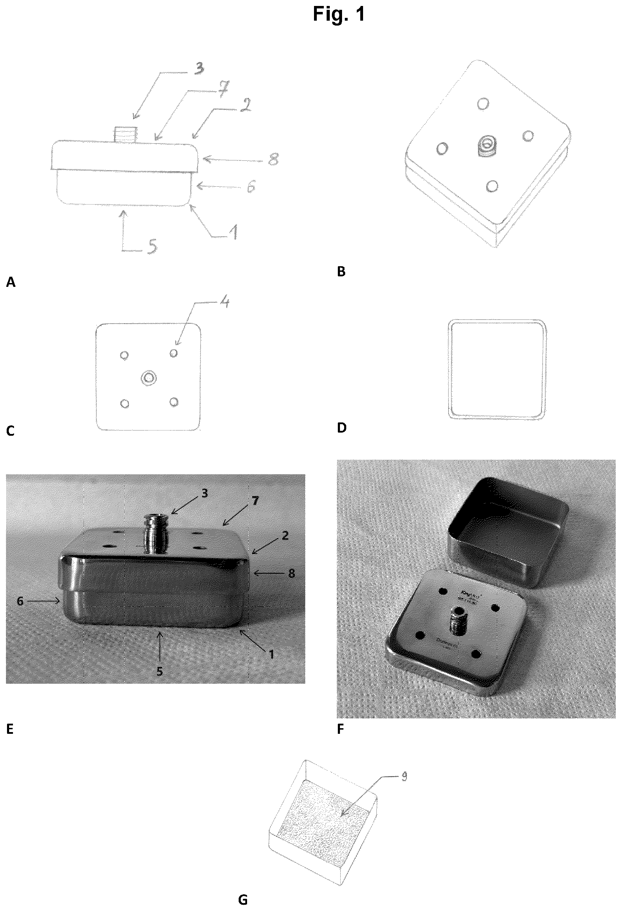

**Fig. 1** shows a set for producing an autologous dressing with a shaping container and cover that is substantially square in cross-section with rounded edges, with 4 holes in the cover. **A** - set assembled in side view, **B** - set assembled in a top view diagonally, **C** - set assembled in top view, **D** - set assembled in bottom view, **E** - photo of the set in a top side view, **F** - photo of the set in unassembled form, **G** - shaping container from above diagonally with a rough surface at the bottom of the interior of the shaping container.

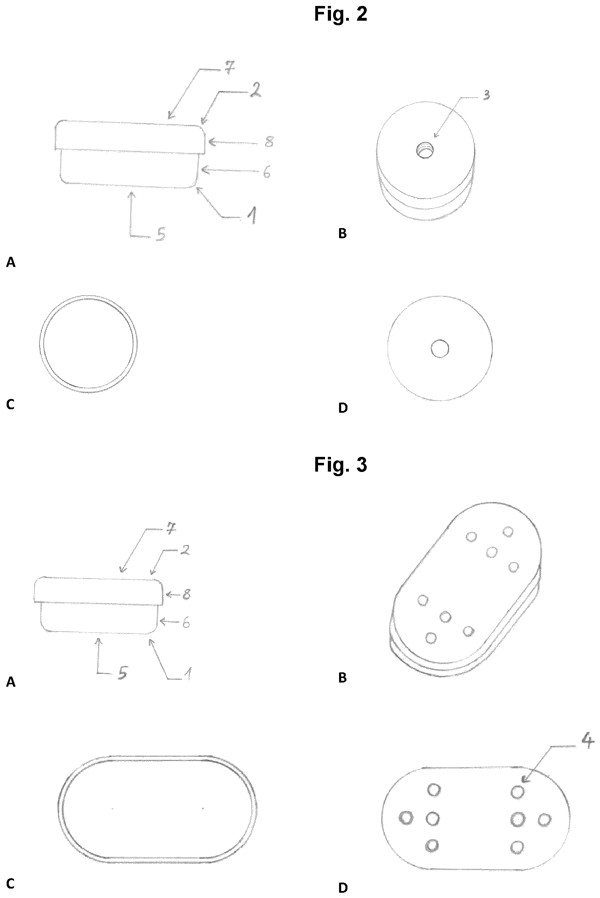

**Fig. 2** schematically shows an exemplary set for producing an autologous dressing in the shape of a shaping container and a cover in the shape of a circle in cross-section, with one hole in the cover. **A** - set assembled in side view, **B** - set assembled in a top view diagonally, **C** - set assembled in bottom view, **D** - set assembled in top view.
**Fig. 3** schematically shows an exemplary set for producing an autologous dressing of a shaping container and an ellipse-shaped cover in cross-section, with 8 holes in the cover. **A** - set assembled in side view, **B** - set assembled in a top view diagonally, **C** - set assembled in bottom view, **D** - set assembled in top view.

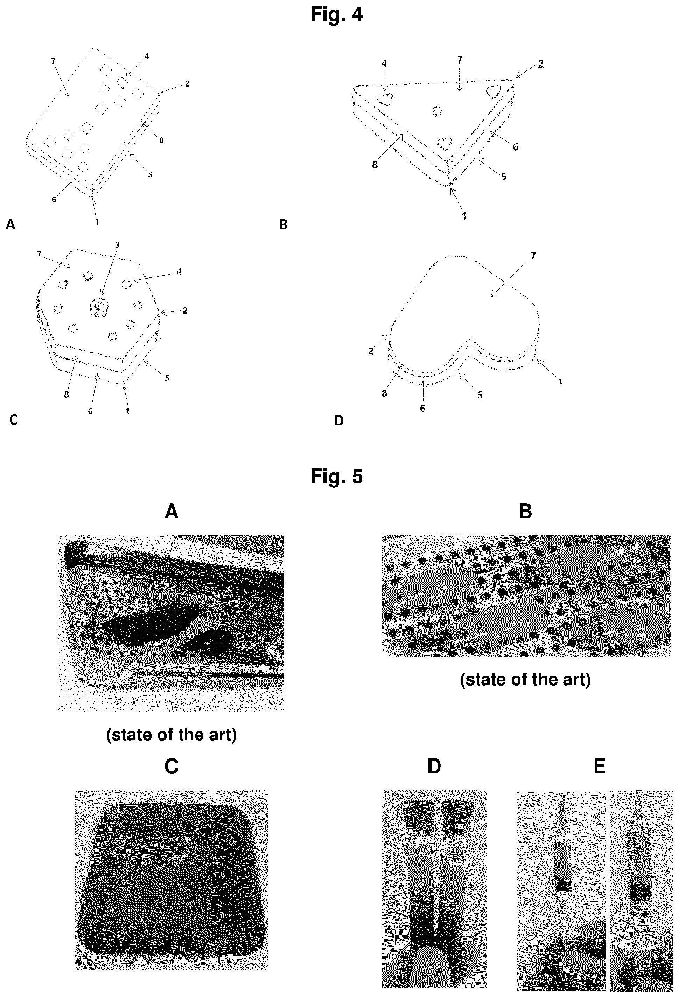

**Fig. 4** schematically shows assembled sets for producing autologous dressings with various shapes of shaping container and cover. **A** - rectangular with 12 square holes in top side view, **B** - triangular in top side view with an inlet for insertion and three triangular holes, **C** - hexagonal in top side view with an inlet for insertion and eight holes in the cover, **D** - heartshaped, without a hole and without an inlet for insertion, in a top side view.
**Fig. 5** presents photos appropriately: **A** and **B** - blood dressings prepared according to a method from the state of the art, **C** - blood dressing prepared by the method of the invention (before removal from the shaping container) , **D** - tubes after blood centrifugation with the I-PRF fraction (left) and the S-PRF fraction (right) before combining them, **E** - syringes with the I-PRF (left) and S-PRF (right) fractions collected after centrifugation before being placed in the shaping container.

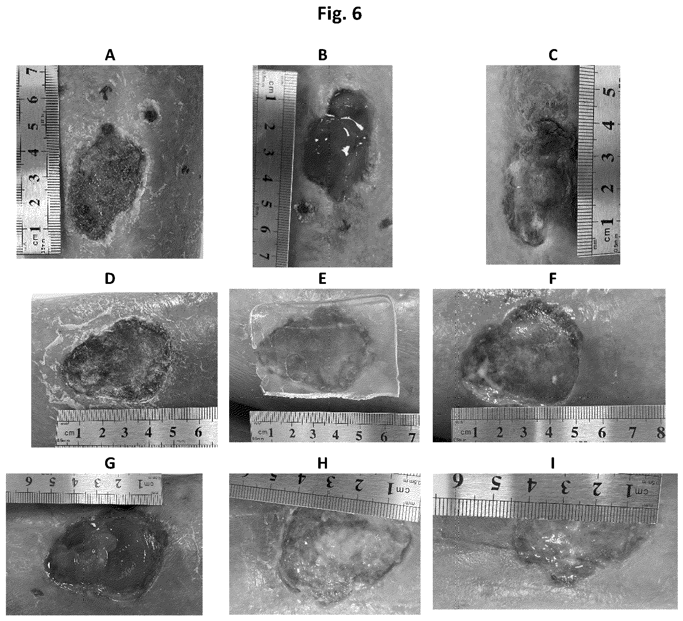

**Fig. 6** presents photos from each phase of the treatment of ulcerative wounds: **A -** wound without dressing in D1 (trial 1), **B** - wound in D1 after applying an autologous dressing (trial 1), **C** - wound in D49 treated with an autologous dressing after its removal (trial 1),
   **D** - wound without dressing in D1 (trial 2a), **E** - wound in D1 after applying the Aqua-Gel^{®} dressing (trial 2a), **F** - wound in D7 after removing the Aqua-Gel^{®} dressing (trial 2a), **G** - wound in D7 after applying an autologous dressing (trial 2b), **H** - wound in D14 after removing the autologous dressing (trial 2b), **I** - wound in D49 after removing the autologous dressing (trial 2b).

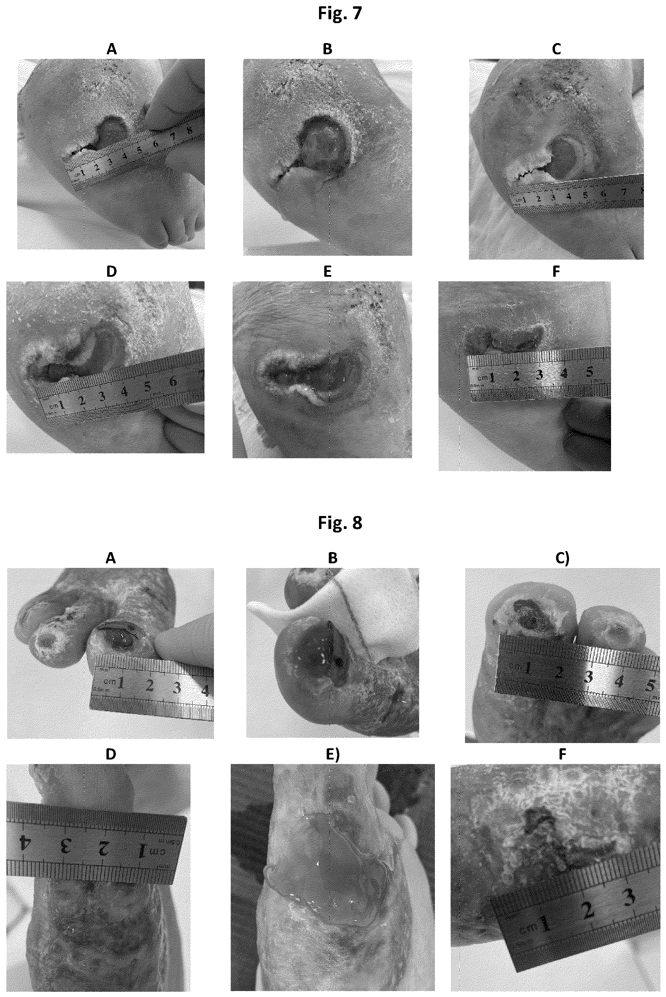

**Fig. 7** presents photos from the individual stages of pressure wound treatment: **A** - wound without dressing in D1 (trial 3), **B** - wound in D1 after applying an autologous dressing (trial 3), **C** - wound in D7 treated with an autologous dressing after its removal (trial 3), **D** - wound in D28 treated with an autologous dressing after its removal (trial 3), E - wound in D28 after applying an autologous dressing (trial 3), **F** - wound in D49 after the removal of the autologous dressing (trial 3).
**Fig. 8** presents photos from the individual phases of burn wound treatment: **A** - wound without dressing in D1 (trial 4), **B** - wound in D1 after applying an autologous dressing (trial 4), **C** - wound in D21 after removal of the autologous dressing (trial 4), **D** - wound without dressing in D1 (trial 5), **E** - wound in D1 after applying an autologous dressing (trial 5), **F** wound in D21 after removal of the autologous dressing (trial 5).

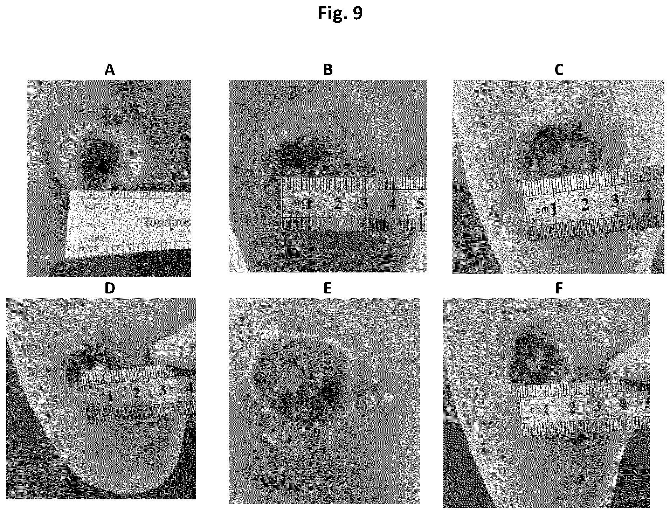

**Fig. 9** presents photos from each phase of the treatment of a diabetic foot wound: **A** - wound without dressing in D1 (trial 6), **B** - wound in D7 treated with an autologous dressing after its removal (trial 6), **C** - wound at D21 treated with an autologous dressing after its removal (trial 6), **D** - wound at D28 treated with an autologous dressing after its removal (trial 6), **E** - wound in D28 after applying an autologous dressing (trial 6), **F** - wound in D35 after removal of the autologous dressing (trial 6).

### EMBODIMENTS OF THE INVENTION

The publications cited in the description and the references provided therein are hereby incorporated by reference in their entirety. The following examples illustrate the invention without limiting it in any way. In the examples standard materials and methods were used according to the manufacturers' recommendations unless otherwise indicated.

### Example 1. Set for producing autologous wound dressings

The set for producing autologous dressings shown in **Fig. 1 A-F** consists of a shaping container 1 and a cover 2 matched both in shape and size, with a cross-sectional shape essentially similar to a square with rounded square vertices. The shaping container 1 has a substantially flat base 5 with a size of 50x50 mm. The side walls 6 of the shaping container have a height of 19 mm. When assembled, the set is 20 mm high. The cover 2 has a lid 7 with a cross-sectional shape similar to a square with rounded tops, with an external size of 52x52 mm. The side walls 8 of the cover are 10 mm high. The cover 2 is sized to fit the shaping container 1 so that when the cover is placed on the shaping container, the side walls of the cover substantially adhere on the inside to the outside of the side walls of the shaping container. The cover 2 has four evenly spaced holes 4 with a diameter of 4 mm each and an inlet for insertion 3, produced as a luer thread for screwing on a syringe with autologous material from the patient. The inlet for insertion 3 has a height of 7 mm, an external diameter of 4 mm and an internal diameter of 3 mm. The luer-lock inlet has a thread and allows the syringe to be screwed onto the inlet for insertion and ensures easy introduction of the patient's autologous material from a syringe with a luer-lock tip, as well as ensures hygiene and sterility when introducing the material into the assembled set.

In this example, the entire set is made of stainless steel. At least the inner surface 9 of the bottom of the shaping container 1 has a rough structure in the range of 0.2 to 0.4 micrometers, wherein the roughness is produced by sandblasting the inner surface of the container. In the case of the set shown in **Fig. 1 A-F**, the roughness is on the entire inner surface of the shaping container and the cover 2. It is advantageous if the roughness is created only on the inner surface 9 of the bottom of the shaping container and the surface of the side walls of the shaping container is smooth, because the autologous dressing produced in such a shaping container is easier to remove from it as a whole **(****Fig. 1G****).**

The set for producing autologous dressings allows the production of an autologous dressing with a surface and shape corresponding to the inner surface of the shaping container 1 and with a dressing thickness ranging from 0.5 to 10 mm, preferably in the range from 1 to 5 mm, more preferably in the range from 2 to 4 mm, depending on the amount of biological material used to produce it.

A ready-to-manufacture set for producing an autologous wound dressing is obtained by placing the cover 2 on the shaping container 1.

### Example 2. N-series of types of sets with various shapes

N-series of types of sets for producing autologous dressings with a fitted shape and size of the shaping container and cover with a cross-section similar to a square **(****Fig. 1 A-F**), a circle **(****Fig. 2A-D**), an ellipse **(****Fig. 3 A-D**), a rectangle **(****Fig. 4A****),** triangle **(****Fig. 4B****),** hexagon **(****Fig. 4C****),** heart shape **(****Fig. 4D****),** were made in the size resulting from the n-series of types of a given shape according to the following schematic description and their representation in the above-mentioned figures. The sets are made of stainless steel.

Each set in a given N-series of types differs in size and consists of a matching shape and size shaping container 1 and cover 2, as described in Example 1, with the difference that:
- the set in **Fig. 4D** does not have a hole 4 nor the inlet for insertion 3;
- in the assembly of **Fig. 4A** there is a number of holes 4, any of which acts as an inlet for insertion 3;
- in the set in **Fig. 2****,** the insertion inlet 3 is an ordinary hole and at the same time it allows for easy sterilization, i.e. it performs the functions of holes 4 in other embodiments.

In the sets, the shaping container 1 has a substantially flat base 5 and the side walls 6 of the shaping container are of substantially equal height. The cover 2 has a lid 7 and cover side walls 8 of substantially equal height. The cover 2 is sized to fit the shaping container 1 so that when the cover is placed on the shaping container, the side walls of the cover substantially adhere on the inside to the outside of the side walls of the shaping container. The height of the side walls 6 of the shaping container is higher than the height of the side walls 8 of the cover, so that when the cover 2 is placed on the shaping container 1, the side walls 6 of the shaping container are not completely covered by the side walls 8 of the cover. When there is at least one hole 4 and/or an inlet 3 for insertion in the cover, it is possible to directly introduce the patient's autologous material into the assembled set, e.g. with a syringe, either through the hole or through the inlet for insertion. Preferably, the inlet for insertion 3 is produced as a luer or luer-lock thread.

**Table 1** below shows the sizes of the various autologous dressing fabrication sets produced.

**Table 1**

| Shape | Size of the shaping base [cm] | Cover size [cm] | Height of the set when assembled [cm] | Number of holes in the cover | Inlet for insertion |
|---|---|---|---|---|---|
| square | 2x2 (sides) | 2.1x2.1 (sides) | 2 | 2 | without thread |
| | 7.5x7.5 (sides) | 7.6x7.6 (sides) | 2.5 | 4 | luer-lock type |
| | 10x10(sides) | 10.1x10.1 (sides) | 4 | 6 | luer-lock type |
| rectangle | 2x6 (sides) | 2.1x6.1 (sides) | 2 | 1 | without thread |
| | 3x8 (sides) | 3.1x8.1 (sides) | 3 | 2 | luer-lock type |
| | 4x10 (sides) | 4.1x10.1 (sides) | 5 | 4 | luer-lock type |
| ellipse | 3x7 (in the minor and major axis) | 3.1x7.1 (in the minor and major axis) | 2 | 2 | luer-lock type |
| | 2x6 | 2.1x6.1 | 5 | 3 | without thread |
| | (in the minor and major axis) | (in the minor and major axis) | | | |
| | 7x15 (in the minor and major axis) | 7.1x15.1 (in the minor and major axis) | 6 | 5 | luer-lock type |
| circle | 3 (diameter) | 3.1 (diameter) | 2 | 2 | luer-lock type |
| | 6 (diameter) | 6.1 (diameter) | 4 | 4 | without thread |
| | 20 (diameter) | 20.1 (diameter) | 10 | 8 | luer-lock type |

### Example 3. Method of production of an autologous dressing

The production of an autologous dressing of the invention was carried out using the following materials and equipment: a laboratory centrifuge with centrifugation in the range ensuring centrifugation of 60-720xg, 16 cm anatomical tweezers, apyrogenic tubes intended for the processing of biological material, a set for producing an autologous dressing made according to **Example 1,** butterfly needle with holder, collection needle, 5 ml syringe, modeling spatula bent on both sides.

### Blood collection and fractionation

Blood was collected from the patient for whom an autologous dressing was to be produced, in a total amount of 18 ml and divided into two tubes of approximately 9 ml each, using a butterfly needle with a holder. Each sample was centrifuged in a different centrifuge, although it is possible to centrifuge the tubes one by one in the same centrifuge with different centrifugation conditions.

To obtain the cell-rich fibrin fraction (I-PRF), the blood sample was centrifuged at 860 RPM (60xg) for 4 minutes, which led to obtaining the I-PRF fraction in the upper layer of the tube. To obtain the structural fibrin fraction (S-PRF), the second tube was centrifuged at 2880 RPM (720xg) for 12 min, which led to obtaining the S-PRF fraction in the upper layer of the sample. After centrifugation, approximately 1.5 ml of the I-PRF fraction and 4 ml of the S-PRF fraction were obtained. Both I-PRF and S-PRF after centrifugation are in the supernatant, i.e. they constitute the upper fraction of centrifuged blood, easy to distinguish and separate, because the lower fraction are erythrocytes, which sink to the bottom under the influence of centrifugation, as shown in **Fig. 5** **D.** To obtain the I-PRF cellular fibrin fraction, centrifugation is performed at 50 RCF (xg) to 70 RCF (xg) for 3 to 5 minutes. To obtain the S-PRF structural fibrin fraction, centrifugation is performed at 600 RCF (xg) to 800 RCF (xg), for 8 to 16 minutes.

### Production of an autologous dressing

Using a syringe with a needle, all the obtained platelet-rich plasma material was collected from the supernatant of both test tubes, i.e. I-PRF and S-PRF fractions **(****Fig. 5E****),** the syringes were screwed one by one onto the luer-lock opening on the assembled autologous dressing set prepared according to **Example 1 (****Fig. 1 A-E**) and the plasma fraction mixture was transferred using a syringe into the set for producing autologous tissue dressings. The whole mixture thereof was gently mixed and set aside for 20 minutes (preferably in the range of 10-40 minutes, more preferably in the range of 15-30 minutes) until the dressing was formed in the form of a gel (this time is an individual matter depending on the patient and the amount of material collected, therefore it may be extended or shortened).

Alternatively, the I-PRF and S-PRF fractions can be combined in one syringe and introduced into the shaping container 6 of a set for producing autologous tissue dressings from blood.

The tests performed show that to produce approximately 1±20% cm³ of a dressing with a standard thickness of 1±20% mm, approximately 2.1-2.3 ml, most often approximately 2.15-2.25 ml of autologous material (combined cellular fibrin I-PRF and structural fibrin S-PRF) is needed. The best results are obtained when using a ratio of 1:5±20%, more preferably 1.5:4±20% of the I-PRF fraction to the S-PRF fraction.

For a dressing measuring 5 cm long x 5 cm wide x 0.1 cm thick, 1.5 ml of centrifuged supernatant of cell-rich fibrin (I-PRF) from one test tube and 4 ml of centrifuged supernatant of structural fibrin (S-PRF) from the second test tube were used, that is, a total of 5.5 ml of autologous material from both fibrin fractions, used to obtain an autologous dressing. After conversion, the result is approximately 2.15-2.25 ml of autologous material in the form of combined cellular fibrin I-PRF and structural fibrin S-PRF) per cubic centimeter of volume for a dressing with a standard thickness of approximately 0.1 cm.

Using the method according to the invention, 1 ml of initial blood produces a dressing with a thickness of approximately 1 mm and a volume of approximately 0.14 cm³ (only 7.14 ml of initial blood is needed to produce 1 cm³ of dressing).

### Applying a dressing to the wound

The prepared dressing had a thickness of about 1 mm, which was then separated from the side walls 6 of the shaping container 1 of the set for producing an autologous dressing from blood using a double-sided bent spatula after slightly lifting the dressing. The finished dressing was applied to the wound by pulling it out of the container using a spatula and the anatomical tweezers included in the set, grabbing the corner of the dressing. The dressing prepared according to the invention was applied to the wound and optionally additionally secured with sterile gauze and/or bandage and/or plaster.

### Preparation of comparative blood dressings to compare performance with the dressing of to the invention

As part of the comparative research, 10 ml of blood was prepared:
**A)** Fibrin clot prepared by the known method
   A dressing was prepared from 10 ml of blood according to the method described in Ghanaati et al., 2014. 10 ml of whole blood was collected. To obtain the cellular fibrin fraction (A-PRF), the blood sample was centrifuged at 1500 RPM for 14 minutes. After centrifugation, the clots were carefully removed from the tubes **(****Fig. 5A****).** The red blood cell (RBC) fraction was removed in such a way as not to damage the bottom of the fibrin-rich clot. Fibrin-based clots with a buffy coat and some red blood cells were fixed with a 4% paraformaldehyde solution for 24 hours. Then they were placed along the longitudinal axis in the mounting cassettes. By pressing the clots into the embedding cassette, a dressing of approximately 3.5 cm long x 1 cm wide x 0.1 cm thick was obtained **(****Fig. 5B****).** In this way, it is possible to obtain a 1 mm thick dressing with a size of approximately 0.35 cm³ from 10 ml of initial blood. To produce 1 cm³ of dressing, 28.57 ml of starting blood is needed.
**B)** By producing a dressing using the method according to the invention from 10 ml of initial blood, a dressing with a thickness of 1 mm and a size of approximately 1.39 cm³ is obtained (only 7.14 ml of initial blood is needed to produce 1 cm³ of the dressing).

The production of an autologous dressing using the method according to the invention, using a dedicated set, provides higher efficiency in terms of the volume and surface of the dressing from the same amount of initial blood.

### Example 4. The use of autologous dressing in wound therapy

The effectiveness of autologous dressings produced according to the invention was confirmed in a clinical comparative study of wound healing in various diseases. Autologous dressings were made from the patients' own blood according to **Example 3.** Subsequent days of treatment were marked as D with the appropriate number of the treatment day.

### A. Wound treatment in chronic leg ulcers

A comparative study was conducted in a 72-year-old patient with non-healing wounds on both legs. Patient with: chronic ulcer of the left and right lower limbs of a mixed type, stent implantation in the left femoral artery, atherosclerosis of the lower limbs, varicose veins of the lower limbs, arterial hypertension and prostatic hypertrophy. The wounds occurred 9 months before inclusion in the clinical trial and had previously been unsuccessfully treated in a surgical clinic with silver dressings, Granuflex, and hydrocolloid dressings. On the start day of the examination, painful wounds with inflammatory exudate.

On the first day D1 of treatment, the wound on the left leg (trial no. 1) was covered with an autologous dressing, and the wound on the right leg with a sterile Aqua-Gel^{®} hydrogel dressing (Kikgel, Poland). The dressing is a transparent sheet of hydrogel with a thickness of approx. 3.5 mm (+/- 0.5 mm), which is an aqueous composition of cross-linked natural and synthetic polymers, producing a three-dimensional, spatial network, with a water content of over 90%, (positive control to D7- trial 2a).

After 7 days of treatment (D7), both dressings were removed. Significant improvement was observed for the wound treated with the autologous dressing, and significant deterioration of the wound condition was observed for the wound treated with the Aqua-Gel^{®} dressing, therefore, for humanitarian reasons, the wound on the right leg was included in the study as a research sample and an autologous dressing was applied to both wounds, both on the left and right leg, at D7. After another week at D14 after starting treatment, significant improvement and reduction of wounds were observed on both limbs. Dressings were changed at seven-day intervals until day 49 (D49). The results obtained are presented in **Table 1,** and photos from the individual stages of treatment of both wounds are shown in **Fig. 6 A-I**.

### B. Pressure sores treatment

The study was carried out in an 18-year-old patient with a pressure sore on the back of the right foot, caused by constant pressure of a given place on the ground. Patient with: cerebral palsy, right clubfoot, pressure sore on the back of the right foot caused by constant pressure on the ground. In the past, he underwent surgery to correct his left foot. The patient tends to have poor wound healing and keloid scars. The wound occurred 12 months before inclusion in the clinical trial and had previously been unsuccessfully treated in a surgical clinic with silver dressings, Granuflex, and hydrocolloid dressings. The patient spent approximately 12 thousands PLN on dressings in the year before treatment.

On the day of starting treatment, the wound was severely swollen on the back of the foot, painful, red, with inflammatory exudate.

On the first day D1 of treatment, the wound on the right leg (trial no. 3) was covered with an autologous dressing. After 7 days (D7), the dressing was removed. Significant improvement was observed for the wound with autologous dressing. After another week on D14 after starting treatment, significant improvement and shrinkage of the wound was observed. Dressings were changed at seven-day intervals until day 49 (D49). The obtained results are presented in **Table** 1, and photos from individual stages of wound treatment are shown in **Fig. 7 A-F**.

### C. Burn wounds treatment

The study was performed on a 23-year-old patient with open wounds on both feet resulting from burns that prevented him from walking normally. Patient with: burns on 19% of the body surface, resulting from loss of consciousness in the shower and the long-term effect of hot water on the body. The burns covered both hands, the right forearm, both feet and the right lower leg. The patient was treated at the Jelenia Góra Valley Hospital, from where he was transported by helicopter to the burns department in Nowa Sól, where he spent 3 months. Numerous skin grafts were performed from the thighs to the lower legs and feet. The wounds occurred 3 months before inclusion in the clinical trial and had previously been unsuccessfully treated with hydrogel dressings, silver dressings, Granuflex, and hydrocolloid dressings. On the day the examination began, the patient had open wounds on both feet, with significant local pain, redness and swelling.

On the first day D1 of treatment, the wound on the right leg (trial no. 4) and the wound on the left leg (trial no. 5) were covered with an autologous dressing. After 7 days (D7), the dressing was removed. Significant improvement was observed for both wounds with autologous dressing. After another week on D14 after starting treatment, significant improvement and shrinkage of the wound was observed. The pain and inflammation disappeared. Dressings were changed at seven-day intervals until day 21 (D21). The obtained results are presented in **Table 1,** and photos from individual stages of wound treatment are shown in **Fig. 8 A-F**.

### D. Diabetic wound treatment - diabetic foot wound

The examination was performed on a 48-year-old patient with a diabetic wound in the right foot. Patient with: obesity, varicose veins, post-thrombotic syndrome, type 2 diabetes, undergoing insulin therapy. The wound occurred 9 months before inclusion in the clinical trial and had previously been repeatedly cleansed by the surgeon from necrotic tissue and keratinized epidermis, and was unsuccessfully treated in the surgical clinic with silver dressings, Granuflex, and hydrocolloid dressings. On the day the examination began, the wound was painful with swelling around the wound and significant inflammatory exudate.

On the first day D1 of treatment, the wound on the right leg (trial no. 6) was covered with an autologous dressing. After 7 days (D7), the dressing was removed. Significant improvement was observed for the wound with autologous dressing. After another week on D14 after starting treatment, significant improvement and shrinkage of the wound was observed. Dressings were changed at seven-day intervals until day 35 (D35). The obtained results are presented in **Table 1,** and photos from the individual phases of wound treatment are shown in **Fig. 9 A-F**.

**Tables 1-3** below present the results obtained for clinical trials from points A-D.

**Tab.1. Measurements of the length, width and depth of the wound in patients with wounds such as venous ulcer, pressure sore, burn and diabetic foot treated with the use of an autologous dressing according to the invention (from the tests in points A-D).**

| **Disease** | **Type of test** | **Test number** | **Dressing applied** | **Experiment day** | **Length [mm]** | **Width [mm]** | **Depth [mm]** |
|---|---|---|---|---|---|---|---|
| Ulcer | Study | 1 | Autologous dressing | 1 | 45 | 28 | 2 |
| | | | | 49 | 28 | 14 | 0 |
| Ulcer | Control | 2a | Aqua-Gel | 1 | 46 | 38 | 3 |
| | | | | 7 | 50 | 44 | 3 |
| Ulcer | Study | 2b | Autologous dressing | 7 | 50 | 44 | 3 |
| | | | | 56 | 36 | 28 | 0 |
| | Study | 3 | | 1 | 49 | 25 | 3 |
| Pressure sore | | | Autologous dressing | 49 | 15 | 5 | 0 |
| Burn | Study | 4 | Autologous dressing | 1 | 10 | 7 | 1 |
| | | | | 21 | 4 | 2 | 0 |
| Burn | Study | 5 | Autologous dressing | 1 | 27 | 13 | 1 |
| | | | | 21 | 9 | 3 | 0 |
| Diabetic foot | Study | 6 | Autologous dressing | 1 | 15 | 13 | 1 |
| | | | | 35 | 3 | 3 | 0 |

**Tab.2. Percentage change in wound length, width and depth in patients with wounds treated with an autologous dressing according to the invention from the tests in points A-D.**

| Disease | A type of test | Test number | Reduction in length [%] | Reduction in width [%] | Reduction in depth [%] |
|---|---|---|---|---|---|
| Ulcer | Study | 1 | 38 | 50 | 100 |
| Ulcer | Control | 2a | -9 | -16 | 0 |
| Ulcer | Study | 2b | 28 | 36 | 100 |
| Pressure sore | Study | 3 | 69 | 80 | 100 |
| Burn | Study | 4 | 60 | 71 | 100 |
| Burn | Study | 5 | 67 | 77 | 100 |
| Diabetic foot | Study | 6 | 80 | 77 | 100 |

**Tab.3. Percentage value of shortening the length of treatment time in patients participating in a clinical trial of wounds using an autologous dressing of the invention from the studies in points A-D.**

| **A type of test** | **Test number** | **Length of treatment before the test [days]** | **Length of treatment during the study [days]** | **Reducing the length of treatment time [%]** |
|---|---|---|---|---|
| Study | 1 | 274 | 49 | 82 |
| | | | | |
| Study | 2b | 274 | 49 | 82 |
| Study | 3 | 365 | 49 | 87 |
| Study | 4 | 91 | 21 | 77 |
| Study | 5 | 91 | 21 | 77 |
| Study | 6 | 274 | 35 | 87 |

The use of autologous dressings produced according to the invention resulted in a significant reduction in the length, width and depth of wounds in each of the studied groups of patients with ulcerative wounds, pressure sores, burns and diabetic foot wounds. **(Tab.1** and **Tab. 2).** In the case of a patient with chronic leg ulcers, the wound length decreased on average by 33% and the width by 43%. In the patient with a pressure sore, the length and width decreased by 69 and 80%, respectively, from baseline. In turn, in a patient with a burn, the wound length decreased on average by 63.5% and the width by 74%. In the case of a patient with diabetic foot, a reduction in wound length by 80% and wound width by 77% was observed. For each of the studied groups of patients, a complete reduction in the depth of wounds was noted. Before the patients began the examination, each wound was characterized by significant local pain, redness, swelling, and in many cases also by exudate. Already one week after the first use of the autologous dressing, patients experienced local pain relief and decreased swelling and redness of the wounds. The use of autologous dressings according to the invention significantly shortened the wound healing time in each of the studied groups **(Table 3).** Burn wounds had the shortest treatment time, while the treatment process for ulcerative and pressure sore wounds took longer. The reduction in treatment time ranged from 77% in a patient with a burn wound to 87% in patients with a pressure sore and diabetic foot. In a patient with chronic venous leg ulcers, an 82% reduction in treatment time was observed compared to the duration of treatment prior to the clinical trial. Before starting the clinical trial, each patient was treated unsuccessfully or with insignificant results with dressings currently available on the market, such as silver dressings, Granuflex, hydrocolloid dressings, Aqua-Gel^{®}, etc., which lasted up to 12 months in a patient with a pressure sore wound. The study showed a significant deterioration of the ulcerative wound condition and an increase in its dimensions after the use of the Agua-Gel^{®} dressing, i.e. in trial 2a **(Table 2** and **Fig. 6E****).** After applying the control dressing (trial 2a), the wound length increased by 9% and the width by 16%, compared to the initial state, without changing the depth thereof. The use of autologous dressings made from the patient's own blood allowed for a reduction in the wound healing time by at least 77% in each of the studied groups of patients, which is a breakthrough in the treatment of various types of wounds and may be the basis for further research on autologous dressings and their use in ulcerative, pressure sore, burn and postoperative wounds, diabetic foot and wounds occurring in other diseases.

### LIST OF DESIGNATIONS

1 - shaping container
2 - cover
3 - inlet for insertion, preferably of the luer-lock type
4 - hole
5 - base of the shaping container
6 - side walls of the shaping container
7 - the lid of the cover (i.e. the essentially flat part of the cover)
8 - side walls of the cover
9 - rough inner surface of the bottom of the shaping container

### LITERATURE

F. Eisinger, J. Patzelt, and H. F. Langer, "The Platelet Response to Tissue Injury," Front. Med., vol. 5, p. 317, 2018.
P. Martin and S. J. Leibovich, "Inflammatory cells during wound repair: the good, the bad and the ugly," Trends Cell Biol., vol. 15, no. 11, pp. 599-607, 2005.
S. Ghanaati et al., "Advanced platelet-rich fibrin: a new concept for cell-based tissue engineering by means of inflammatory cells.," J. Oral Implantol., vol. 40, no. 6, pp. 679-689, Dec. 2014.

## Claims

1. A set for producing an autologous dressing from blood, **characterized in that** it comprises a shaping container (1) and a cover (2) of the shaping container with a shape and size resulting from the n-series of types;
the shaping container (1) has a substantially flat base (5) and side walls (6) of the shaping container of substantially equal height;
the cover (2) has a lid (7) and side walls (8) of the cover of substantially equal height;
the cover (2) is matched both in shape and size to the shaping container (1) so that when the cover is placed on the shaping container, the side walls of the cover substantially adhere by the inner side thereof to the outer side of the side walls of the shaping container;
the side walls (6) of the shaping container are higher than the side walls (8) of the cover;
the shaping container (1) is made of stainless steel and at least the bottom surface of the shaping container has a rough surface (9) from the inside, with a roughness degree in the range of 0.01-100 µm.

2. The set according to claim 1, **characterized in that** both the shaping container (1) and the cover (2) of the shaping container are made of stainless steel, preferably of stainless steel approved for medical use.

3. The set according to claims 1-2, **characterized in that** at least one hole (4) and/or an inlet for insertion (3) is provided in a cover (2);
wherein preferably the inlet for insertion (3) is of the luer-lock or luer type;
wherein preferably, the shaping container (1) and the cover (2) of the shaping container in the n-series of types are selected from a shape with a cross-section with a shape similar to a square, circle, ellipse, rectangle, triangle, hexagon, heart shape, preferably a square with rounded vertices.

4. The set according to claims 1-3, **characterized in that** the roughness degree is in the range of 0.05-10 µm, more preferably in the range of 0.2-0.4 µm;
wherein preferably the rough surface is a surface of the sandblasted type, diamond dust type, a surface with linear porosity, a surface with cross-porosity;
wherein preferably the rough surface is produced by sandblasting, by producing micro-cuts and/or micro-cavities, preferably the micro-cuts and/or micro-cavities are produced by laser or mechanically.

5. The set according to claims 1-4, **characterized in that** the set has a cross-sectional shape similar to a square with rounded vertices, an external size of 52x52±20% mm, a side wall (8) of the cover with a height of 10±20% mm; there are four holes (4) evenly spaced in the cover (2) and a central inlet for insertion (3) of the luer-lock type, wherein both the shaping container (1) and the cover (2) are made of stainless steel, wherein the inner bottom surface (9) of the shaping container (1) has a rough structure in the range from 0.2 to 0.4 µm, more preferably, the entire inner surface of the shaping container (1) has a rough structure with a roughness degree from 0.2 to 0.4 µm.

6. A method of production of an autologous dressing from the blood of a subject, **characterized in that** it comprises steps, in which:
a) the blood collected from the subject is divided into two volumes, preferably two substantially equal volumes;
- from the first volume, the cell-rich fibrin fraction (I-PRF) is obtained by centrifugation from 50 RCF (xg) to 70 RCF (xg) for 3 to 5 minutes;
- from the second volume, the S-PRF structural fibrin fraction is obtained by centrifugation at 600 RCF (xg) to 800 RCF (xg) for 8 to 16 minutes;
b) the I-PRF and S-PRF fractions obtained in a) are introduced into the shaping container (6) of the set for producing autologous tissue dressings from blood as defined in claims 1-4;
c) to produce an autologous dressing, the set for producing autologous tissue dressings with combined I-PRF and S-PRF fractions is set aside for the time needed to prepare the gel form, preferably for 5 to 240 minutes.

7. The method according to claim 6, **characterized in that** in step c) the set for producing autologous tissue dressings with combined I-PRF and S-PRF fractions is set aside for a time in the range of 10-40 minutes, more preferably in the range of 15-30 minutes;
wherein preferably in step c), the combined I-PRF and S-PRF fractions are gently mixed together before the set is set aside to produce an autologous tissue dressing, preferably by gently rocking the shaping container (6) of the set.

8. The method according to claims 6-7, **characterized in that** in step b) the I-PRF and S-PRF fractions are combined in a ratio of 1:5±20% of the I-PRF fraction to the S-PRF fraction, more wherein preferably 1.5:4±20% of the I-PRF fraction to the S-PRF fraction;
wherein preferably in step b) the I-PRF and S-PRF fractions are combined in a syringe, preferably in a syringe equipped with a luer-lock type tip;
preferably in step b), the combined I-PRF and S-PRF fractions are introduced into the shaping container (6) through the hole (4) in the cover (2) of the set for producing autologous tissue dressings and/or through the inlet for insertion (3) in the cover (2), preferably introduced through the inlet for insertion (3), preferably introduced through the luer, luer-lock type inlet.

9. The method according to claims 6-9, **characterized in that** in step a) the separation to the cell-rich fibrin fraction (I-PRF) and the structural fibrin fraction (S-PRF) is carried out by parallel centrifugation in two centrifuges.

10. The method according to claims 6-9, **characterized in that** in step a) the separation into the cell-rich fibrin fraction (I-PRF) and the structural fibrin fraction (S-PRF) is carried out by parallel centrifugation in two centrifuges;
wherein preferably in step a) the separation into the cell-rich fibrin fraction (I-PRF) and the structural fibrin fraction (S-PRF) is carried out by centrifugation of blood in apyrogenic tubes.

11. The method according to claims 6-10, **characterized in that** the subject is a mammal, preferably a human.

12. An autologous dressing from blood produced by the method as defined in claims 6-11.

## Patentansprüche

1. Set zur Herstellung eines autologen Verbandes aus Blut, **dadurch gekennzeichnet, dass** es einen Formbehälter (1) und eine Abdeckung (2) für den Formbehälter umfasst, deren Form und Größe sich aus der n-Typenreihe ergeben;
wobei der Formbehälter (1) einen im Wesentlichen flachen Boden (5) und Seitenwände (6) des Formbehälters im Wesentlichen die gleiche Höhe aufweisen;
wobei die Abdeckung (2) einen Deckel (7) und Seitenwände (8) der Abdeckung im Wesentlichen die gleiche Höhe aufweisen;
wobei die Abdeckung (2) sowohl in der Form als auch in der Größe auf den Formbehälter (1) abgestimmt ist, sodass beim Aufsetzen der Abdeckung auf den Formbehälter die Seitenwände der Abdeckung im Wesentlichen mit der Innenseite davon an der Außenseite der Seitenwände des Formbehälters anliegen;
wobei die Seitenwände (6) des Formbehälters höher als die Seitenwände (8) der Abdeckung sind;
wobei der Formbehälter (1) aus Edelstahl besteht und zumindest die Bodenoberfläche des Formbehälters von innen eine raue Oberfläche (9) mit einem Rauheitsgrad im Bereich von 0,01-100 µm aufweist.

2. Set nach Anspruch 1, **dadurch gekennzeichnet, dass** sowohl der Formbehälter (1) als auch die Abdeckung (2) des Formbehälters aus Edelstahl bestehen, bevorzugt aus für medizinische Zwecke zugelassenem Edelstahl.

3. Set nach den Ansprüchen 1-2, **dadurch gekennzeichnet, dass** in einer Abdeckung (2) zumindest ein Loch (4) und/oder eine Einführöffnung (3) vorgesehen ist;
wobei bevorzugt die Einführöffnung (3) vom Luer-Lock- oder Luer-Typ ist;
wobei bevorzugt der Formbehälter (1) und die Abdeckung (2) des Formbehälters in der n-Typenreihe aus einer Form mit einem Querschnitt ausgewählt sind, dessen Form einem Quadrat, einem Kreis, einer Ellipse, einem Rechteck, einem Dreieck, einem Sechseck, einer Herzform, bevorzugt einem Quadrat mit abgerundeten Ecken, ähnelt.

4. Set nach den Ansprüchen 1-3, **dadurch gekennzeichnet, dass** der Rauheitsgrad im Bereich von 0,05-10 µm, besonders bevorzugt im Bereich von 0,2-0,4 µm, liegt;
wobei bevorzugt die raue Oberfläche eine Oberfläche vom sandgestrahlten Typ, vom Diamantstaub-Typ, eine Oberfläche mit linearer Porosität oder eine Oberfläche mit kreuzförmiger Porosität ist;
wobei bevorzugt die raue Oberfläche durch Sandstrahlen, durch Erzeugen von Mikroschnitten und/oder Mikrovertiefungen hergestellt wird, wobei bevorzugt die Mikroschnitte und/oder Mikrovertiefungen mittels Laser oder mechanisch hergestellt werden.

5. Set nach den Ansprüchen 1-4, **dadurch gekennzeichnet, dass** das Set eine Querschnittsform, die einem Quadrat mit abgerundeten Ecken ähnelt, eine Außenabmessung von 52 × 52 ± 20 % mm und eine Seitenwand (8) der Abdeckung mit einer Höhe von 10 ± 20 % mm aufweist; wobei sich in der Abdeckung (2) vier gleichmäßig beabstandete Löcher (4) und eine zentrale Einführöffnung (3) vom Luer-Lock-Typ befinden, wobei sowohl der Formbehälter (1) als auch die Abdeckung (2) aus Edelstahl bestehen, wobei die innere Bodenoberfläche (9) des Formbehälters (1) eine raue Struktur im Bereich von 0,2 bis 0,4 µm aufweist, wobei besonders bevorzugt die gesamte Innenoberfläche des Formbehälters (1) eine raue Struktur mit einem Rauheitsgrad von 0,2 bis 0,4 µm aufweist.

6. Verfahren zur Herstellung eines autologen Verbandes aus dem Blut eines Subjekts, **dadurch gekennzeichnet, dass** es folgende Schritte umfasst, bei denen:
a) die von dem Subjekt entnommene Blutprobe in zwei Volumina aufgeteilt wird, bevorzugt in zwei im Wesentlichen gleich große Volumina;
- aus dem ersten Volumen die zellreiche Fibrinfraktion (I-PRF) durch Zentrifugation bei 50 RCF (xg) bis 70 RCF (xg) für 3 bis 5 Minuten gewonnen wird;
- aus dem zweiten Volumen die strukturelle Fibrinfraktion S-PRF durch Zentrifugation bei 600 RCF (xg) bis 800 RCF (xg) für 8 bis 16 Minuten gewonnen wird;
b) die unter a) gewonnenen I-PRF- und S-PRF-Fraktionen in den Formbehälter (6) des Sets zur Herstellung der in den Ansprüchen 1-4 definierten, autologen Gewebeverbänden aus Blut eingeführt werden;
c) zur Herstellung eines autologen Verbandes das Set zur Herstellung autologer Gewebeverbände mit kombinierten I-PRF- und S-PRF-Fraktionen für die zur Herstellung der Gelform erforderliche Zeit beiseitegestellt wird, bevorzugt für 5 bis 240 Minuten.

7. Verfahren nach Anspruch 6, **dadurch gekennzeichnet, dass** in Schritt c) das Set zur Herstellung autologer Gewebeverbände mit kombinierten I-PRF- und S-PRF-Fraktionen für eine Zeit im Bereich von 10-40 Minuten, besonders bevorzugt im Bereich von 15-30 Minuten, beiseitegestellt wird;
wobei bevorzugt in Schritt c) die kombinierten I-PRF- und S-PRF-Fraktionen schonend miteinander vermischt werden, bevor das Set zur Herstellung eines autologen Gewebeverbandes beiseitegestellt wird, bevorzugt durch sanftes Schwenken des Formbehälters (6) des Sets.

8. Verfahren nach den Ansprüchen 6-7, **dadurch gekennzeichnet, dass** in Schritt b) die I-PRF- und die S-PRF-Fraktion in einem Verhältnis von 1:5 ± 20 % der I-PRF-Fraktion zur S-PRF-Fraktion miteinander kombiniert werden, wobei bevorzugt ein Verhältnis von 1,5:4 ± 20 % der I-PRF-Fraktion zur S-PRF-Fraktion vorliegt;
wobei bevorzugt in Schritt b) die I-PRF- und S-PRF-Fraktionen in einer Spritze kombiniert werden, bevorzugt in einer Spritze, die mit einer Luer-Lock-Spitze ausgestattet ist;
wobei bevorzugt in Schritt b) die kombinierten I-PRF- und S-PRF-Fraktionen durch das Loch (4) in der Abdeckung (2) des Sets zur Herstellung autologer Gewebeverbände und/oder durch die Einführöffnung (3) in der Abdeckung (2) in den Formbehälter (6) eingeführt, bevorzugt durch die Einführöffnung (3) eingeführt, bevorzugt durch die Öffnung vom Luer-, Luer-Lock-Typ eingeführt werden.

9. Verfahren nach den Ansprüchen 6-9, **dadurch gekennzeichnet, dass** in Schritt a) die Trennung in die zellreiche Fibrinfraktion (I-PRF) und die strukturelle Fibrinfraktion (S-PRF) durch parallele Zentrifugation in zwei Zentrifugen erfolgt.

10. Verfahren nach den Ansprüchen 6-9, **dadurch gekennzeichnet, dass** in Schritt a) die Trennung in die zellreiche Fibrinfraktion (I-PRF) und die strukturelle Fibrinfraktion (S-PRF) durch parallele Zentrifugation in zwei Zentrifugen erfolgt;
wobei bevorzugt in Schritt a) die Trennung in die zellreiche Fibrinfraktion (I-PRF) und die strukturelle Fibrinfraktion (S-PRF) durch Zentrifugation des Blutes in apyrogenen Röhrchen erfolgt.

11. Verfahren nach den Ansprüchen 6-10, **dadurch gekennzeichnet, dass** das Subjekt ein Säugetier, bevorzugt ein Mensch, ist.

12. Autologer Verband aus Blut, der nach dem in den Ansprüchen 6-11 definierten Verfahren hergestellt wird.

## Revendications

1. Ensemble destiné à la production d'un pansement autologue à partir de sang, **caractérisé en ce qu'**il comprend un récipient de mise en forme (1) et un couvercle (2) du récipient de mise en forme, dont la forme et les dimensions résultent de la série n de types ;
dans lequel le récipient de mise en forme (1) a un fond (5) sensiblement plat et les parois latérales (6) du récipient de mise en forme ont une hauteur sensiblement égale ;
dans lequel le couvercle (2) a un dessus (7) et les parois latérales (8) du couvercle sont de hauteur sensiblement égale ;
dans lequel le couvercle (2) est adapté au récipient de mise en forme (1) tant par la forme que par les dimensions, de sorte que, lorsque le couvercle est placé sur le récipient de mise en forme, les parois latérales du couvercle adhèrent sensiblement, par leur face interne, à la face externe des parois latérales du récipient de mise en forme ;
dans lequel les parois latérales (6) du récipient de mise en forme sont plus hautes que les parois latérales (8) du couvercle ;
dans lequel le récipient de mise en forme (1) est en acier inoxydable et au moins la surface inférieure du récipient de mise en forme a une surface rugueuse (9) à l'intérieur, avec un degré de rugosité compris entre 0,01 et 100 µm.

2. Ensemble selon la revendication 1, **caractérisé en ce que** tant le récipient de mise en forme (1) que le couvercle (2) du récipient de mise en forme sont en acier inoxydable, de préférence en acier inoxydable approuvé pour un usage médical.

3. Ensemble selon les revendications 1 à 2, **caractérisé en ce que** au moins un trou (4) et/ou un orifice d'insertion (3) est prévu dans un couvercle (2) ;
dans lequel, de préférence, l'orifice d'insertion (3) est de type Luer-Lock ou Luer ;
dans lequel, de préférence, le récipient de mise en forme (1) et le couvercle (2) du récipient de mise en forme, dans la série n de types, sont choisis parmi des formes avec une section transversale de forme similaire à un carré, un cercle, une ellipse, un rectangle, un triangle, un hexagone, un cœur, de préférence un carré aux sommets arrondis.

4. Ensemble selon les revendications 1 à 3, **caractérisé en ce que** le degré de rugosité est compris entre 0,05 et 10 µm, de préférence entre 0,2 et 0,4 µm ;
dans lequel, de préférence, la surface rugueuse est une surface de type sablée, de type poussière de diamant, une surface présentant une porosité linéaire ou une surface présentant une porosité transversale ;
dans lequel, de préférence, la surface rugueuse est produite par sablage, en produisant des micro-entailles et/ou des micro-cavités, de préférence les micro-entailles et/ou les micro-cavités étant produites par laser ou par voie mécanique.

5. Ensemble selon les revendications 1 à 4, **caractérisé en ce que** l'ensemble a une forme en coupe transversale similaire à un carré aux sommets arrondis, une dimension extérieure de 52 × 52 ± 20 % mm, une paroi latérale (8) du couvercle d'une hauteur de 10 ± 20 % mm ; il y a quatre trous (4) espacés uniformément dans le couvercle (2) et un orifice d'insertion (3) central de type Luer-Lock, dans lequel tant le récipient de mise en forme (1) que le couvercle (2) sont en acier inoxydable, dans lequel la surface inférieure interne (9) du récipient de mise en forme (1) a une structure rugueuse comprise entre 0,2 et 0,4 µm, de préférence, l'ensemble de la surface intérieure du récipient de mise en forme (1) a une structure rugueuse dont le degré de rugosité est compris entre 0,2 et 0,4 µm.

6. Procédé de production d'un pansement autologue à partir du sang d'un sujet, **caractérisé en ce qu'**il comprend les étapes dans lesquelles :
a) le sang prélevé sur le sujet est divisé en deux volumes, de préférence deux volumes sensiblement égaux ;
- à partir du premier volume, la fraction de fibrine riche en cellules (I-PRF) est obtenue par centrifugation à une vitesse comprise entre 50 RCF (xg) et 70 RCF (xg) pendant 3 à 5 minutes ;
- à partir du deuxième volume, la fraction de fibrine structurelle S-PRF est obtenue par centrifugation à une vitesse comprise entre 600 RCF (xg) et 800 RCF (xg) pendant 8 à 16 minutes ;
b) les fractions d'I-PRF et de S-PRF obtenues en a) sont introduites dans le récipient de mise en forme (6) de l'ensemble destiné à la production de pansements tissulaires autologues à partir de sang, tel que défini dans les revendications 1 à 4 ;
c) pour produire un pansement autologue, l'ensemble destiné à la production de pansements tissulaires autologues à base de fractions d'I-PRF et de S-PRF combinées est mis de côté pendant le temps nécessaire à la préparation de la forme gélifiée, de préférence pendant 5 à 240 minutes.

7. Procédé selon la revendication 6, **caractérisé en ce que**, à l'étape c), le mélange destiné à la production de pansements tissulaires autologues à base de fractions d'I-PRF et de S-PRF combinées est laissé au repos pendant une durée comprise entre 10 et 40 minutes, de préférence entre 15 et 30 minutes ;
dans lequel, de préférence, à l'étape c), les fractions d'I-PRF et de S-PRF combinées sont mélangées délicatement avant que l'ensemble ne soit mis de côté pour produire un pansement tissulaire autologue, de préférence en secouant délicatement le récipient de mise en forme (6) de l'ensemble.

8. Procédé selon les revendications 6 à 7, **caractérisé en ce que**, à l'étape b), les fractions d'I-PRF et de S-PRF sont combinées dans un rapport de 1 : 5 ± 20 % de la fraction d'I-PRF par rapport à la fraction de S-PRF, plus préférablement de 1,5 : 4 ± 20 % de la fraction d'I-PRF par rapport à la fraction de S-PRF ;
dans lequel, de préférence, à l'étape b), les fractions d'I-PRF et de S-PRF sont combinées dans une seringue, de préférence dans une seringue munie d'un embout de type Luer-Lock ;
de préférence, à l'étape b), les fractions d'I-PRF et de S-PRF combinées sont introduites dans le récipient de mise en forme (6) par le trou (4) dans le couvercle (2) de l'ensemble destiné à la production de pansements tissulaires autologues et/ou par l'orifice d'insertion (3) dans le couvercle (2), de préférence introduites par l'orifice d'insertion (3), de préférence introduites par l'orifice de type Luer, Luer-Lock.

9. Procédé selon les revendications 6 à 9, **caractérisé en ce que**, à l'étape a), la séparation entre la fraction de fibrine riche en cellules (I-PRF) et la fraction de fibrine structurelle (S-PRF) est effectuée par centrifugation en parallèle dans deux centrifugeuses.

10. Procédé selon les revendications 6 à 9, **caractérisé en ce que**, à l'étape a), la séparation entre la fraction de fibrine riche en cellules (I-PRF) et la fraction de fibrine structurelle (S-PRF) est effectuée par centrifugation en parallèle dans deux centrifugeuses ;
dans lequel, de préférence, à l'étape a), la séparation entre la fraction de fibrine riche en cellules (I-PRF) et la fraction de fibrine structurelle (S-PRF) est effectuée par centrifugation du sang dans des tubes apyrogènes.

11. Procédé selon les revendications 6 à 10, **caractérisé en ce que** le sujet est un mammifère, de préférence un humain.

12. Pansement autologue à partir de sang, produit selon le procédé défini dans les revendications 6 à 11.
